# EUROPÄISCHE PATENTSCHRIFT

(11) **EP 1 490 038 B1**
(45) Veröffentlichungstag und Bekanntmachung des Hinweises auf die Patenterteilung: **05.08.2009**
(21) Anmeldenummer: 03722347.6
(22) Anmeldetag: 21.03.2003
(51) Int. Cl.: A61K 9/70

(54) **MISCHSYSTEM ZUR LÖSLICHKEITSVERMITTLUNG VON PHARMAZEUTISCHEN WIRKSTOFFEN IN POLYMERMATRIZES**
HYBRID SYSTEM FOR SOLUBILIZING PHARMACEUTICALLY ACTIVE SUBSTANCES IN POLYMER MATRICES
SYSTEME DE MELANGE POUR SOLUBILISER DES AGENTS ACTIFS PHARMACEUTIQUES DANS DES MATRICES POLYMERES

(30) Priorität: 22.03.2002 DE 10212864
(43) Veröffentlichungstag der Anmeldung: 29.12.2004
(73) Patentinhaber: Beiersdorf AG, 20245 Hamburg (DE)
(72) Erfinder: NIERLE, Jens, 21147 Hamburg (DE); GÄDE, Christian, 21629 Neu Wulmstorf (DE)
(86) Internationale Anmeldenummer: PCT/EP2003/002953
(87) Internationale Veröffentlichungsnummer: WO 2003/080035

(56) Entgegenhaltungen:
- US-A- 5 702 720
- US-B1- 6 221 383

## Beschreibung

Die vorliegende Erfindung betrifft Mischsysteme umfassend die Komponenten Polyvinylpyrrolidon und/oder deren Copolymere und Dimethylsulfoxid. Weiterhin umfasst die Erfindung Polymermatrizes enthaltend diese Mischsysteme, transdermale therapeutische Systeme auf Basis der Polymermatrizes sowie deren Herstellung. Das Mischsystem ermöglicht, ansonsten schwer lösliche, pharmazeutische Wirkstoffe, z.B. Ibuprofen, in eine unpolare Matrix einzubringen. Der Vorteil des Systems ist, dass damit polare Wirkstoffe in einer unpolaren Matrix wie z.B. Polyisobutylen über den Sättigungspunkt hinaus gelöst werden können. Darüber hinaus verhindert das Mischsystem die Kristallisation des Wirkstoffs, wodurch eine konstante Freisetzung des Wirkstoffs fiber die gesamte Haltbarkeitsdauer eines transdermalen Systems, wie z.B. Pflaster, gewährleistet wird.

Bei der Formulierung von Arzneimitteln zur topischen Applikation von Wirkstoffen kommt der Wahl der Hilfsstoffe entscheidende Bedeutung zu. So ist bekannt, dass ein Zusammenhang zwischen Löslichkeit eines Wirkstoffs in einem Vehikel und seiner Freisetzung aus diesem Vehikel besteht Wird ein wirkstoffhaltiges Vehikel auf die Haut appliziert, dann stellt sich ein Verteilungsgleichgewicht des Wirkstoffs zwischen Vehikel und Haut ein. Die Lage dieses Gleichgewichts wird von der Löslichkeit in beiden Phasen bestimmt.

Transdermal Therapeutische Systeme (TTS) zur Abgabe von Wirkstoffen durch die Haut sind seit langer Zeit bekannt. Die topische Applikation von Arzneimitteln über wirkstoffhaltige Pflastersysteme bietet zwei Hauptvorteile: Erstens wird durch diese Darreichungsform eine Freisetzungskinetik des Wirkstoffes erster Ordnung realisiert, wodurch über einen sehr langen Zeitraum ein konstanter Wirkstoffspiegel im Organismus aufrechterhalten werden kann. Zweitens werden über den Aufnahmeweg durch die Haut der Magen-Darm-Trakt sowie die erste Leberpassage vermieden. Dadurch können ausgewählte Arzneistoffe in einer geringen Dosierung wirkungsvoll verabreicht werden. Dies ist insbesondere dann von Vorteil, wenn eine lokale Wirkung des Arzneistoffes unter Umgehung einer systemischen Wirkung erwünscht ist. Dies ist zum Beispiel bei der Behandlung rheumatischer Gelenkbeschwerden oder Muskelentzündungen der Fall.

Eine in der Fachliteratur gut beschriebene Ausführungsform solcher transdermalen Systeme stellen Matrixsysteme oder monolitische Systeme dar, in denen der Arzneistoff direkt in den druckempfindlichen Haftklebstoff eingearbeitet wird. Eine solche haftklebrige, wirkstoffhaltige Matrix ist im anwendungsfertigen Produkt auf der einen Seite mit einem für den Wirkstoff undurchlässigen Träger ausgestattet, auf der gegenüberliegenden Seite befindet sich eine mit einer Trennschicht ausgestattete Trägerfolie, die vor der Applikation auf die Haut entfernt wird (kleben&dichten, Nr.42, 1998, S. 26 bis 30). So wird vornehmlich die Verwendung von Polyacrylaten und/oder Polyurethanen als Basis der haftklebrigen Polymermatrix erwähnt (Lamba, Woodhouse, Cooper, "Polyurethanes in Biomedical Applications", CRC Press, 1998, S. 240) und WO 01/68060.

Ein Problem bei der Herstellung transdermaler therapeutischer Systeme ist das Einbringen polarer Wirkstoffe in die zumeist unpolaren Polymermatrizes. Dadurch können bevorzugte Wirkstoffe mitunter nur schlecht oder nur in begrenzter Konzentration in die Polymermatrix eingearbeitet werden. Des weiteren besteht die Gefahr, dass aufgrund der unterschiedlichen Polarität und Unlöslichkeit der Wirkstoffe in der Polymermatrix die Wirkstoffe mit der Zeit aus dem Polymersystem auskristallisieren. Eine Langzeitstabilität ist damit nicht gewährleistet.

Einen Vorschlag zur Lösung dieser Probleme wird durch den Zusatz von Lösemittel gegeben. Die Einarbeitung von Lösemittel in eine polymere Matrix hat aber verschiedene Nachteile. Zu erwähnen sind hier die Schädlichkeit der meisten organischen Lösungsmittel, hohe technische Aufwände für Absaugung und Wiedergewinnung, hohe Kosten für erforderliche hochreine Lösungsmittel und besonders ein sehr hoher Aufwand zur Entfernung von Lösungsmittelresten aus der Matrix.

Diese Lösemittel werden ebenfalls durch die Haut resorbiert, wodurch sich der Lösemittelgehalt in der TTS-Matrix verringert. Weiterhin wurde gefunden, dass von der Haut abgegebenes Wasser sich im Lösungsmittel anreichert, da es über die hydrophobe Polymermatrix schlecht gebunden werden kann. Beide Mechanismen führen dazu, dass die Wirkstofffreisetzung ungünstig beeinflusst wird.

WO 01/68060 A2 beschreibt ein transdermales therapeutisches System vom Matrix-Typ, wobei den hydrophoben Basispolymeren der wirkstoffhaltigen Polymermatrix Polyacrylatpolymere beigemischt werden. Die hydrophoben Basispolymere können ausgewählt werden aus der Gruppe der Polysiloxane, Polyisobutylen, Polyisopren, Styrol-Dien-Styrol-Blockcopolymer oder Mischungen daraus. Die Polyacrylatpolymere sollen den Nachteil der Übersättigung des Wirkstoffs im TTS durch die Lösungsmittelzugabe als auch eine Rekristallisierung vor der Aufnahme durch die Haut vermeiden.

WO 94/23713 beschreibt entzündungshemmende Arzneimittel auf Phenylpropionsäure-und Phenylessigsäure-Basis zur topischen Applikation, weiche Kombinationen von lipophilen und hydrophilen Hilfsstoffen enthalten. Über Wirkungsweise und Funktion dieser Hilfsstoffe werden keine Angaben gemacht.

Aufgabe der vorliegenden Erfindung ist es ein System zu entwickeln, das die Aufnahme von polaren Wirkstoffen in unpolaren Polymermatrizes ermöglicht und das Auskristallisieren der Wirkstoffe in den unpolaren Polymermatrizes verhindert ohne dass zusätzliche Lösungsmittel eingesetzt werden.

An wirkstoffhaltige Pflastersysteme werden neben einer kontrollierten Wirkstofffreisetzung aber auch bestimmte Anforderungen an die Klebmatrix, wie beispielsweise Hautfreundlichkeit, gutes Haftvermögen über eine lange Anwendungsdauer und eine schmerzfreie Entfembarkeit gestellt. Eine häufig beobachtete Nebenwirkung insbesondere bei Polyacrylatklebmatrizes ist das Auftreten von Hautirritationen, die besonders bei längerer oder wiederholter Applikation eines wirkstoffhaltigen Pflasters an einer gleichbleibenden Körperregion auftreten. Sie werden hauptsächlich durch die Inhaltsstoffe der haftklebrigen Matrix verursacht. Auch ein schmerzhaftes Wiederablösen des wirkstoffhaltigen Pflasters nach längerer Tragezeit wird häufig beobachtet. Es sollte daher auf den Zusatz weiterer Klebstoffe, die unter Umständen Nebenwirkungen wie Mazeration, Allergien u.a. hervorrufen, verzichtet werden.
Wirkstoffhaltige Pflaster werden in der Regel auf gesunder, intakter Haut appliziert. Gerade hier ist es besonders wichtig, die intakte Haut nicht durch ein Arzneimittel zu reizen oder gar zu schädigen. Eine genügende Kohäsivität ist zusätzlich notwendig, um das wirkstoffhaltige Pflaster nach beendeter Tragedauer rückstandsfrei und insbesondere auch schmerzfrei entfernen zu können.

Aufgabe der vorliegenden Erfindung ist es daher auch ein transdermales therapeutisches System bereit zu stellen, das dem Anwender den bestmöglichen Komfort, d.h. lange Tragedauer, hautschonende Applikation und schmerzfreie Wiederablösung, ermöglicht.

Gelöst werden die angeführten Aufgaben durch ein Mischsystem bestehend aus Polyvinylpyrrolidon (PVP) und/oder deren Copolymere und Dimethylsulfoxid (DMSO), Polymermatrizes enthaltend dieses Mischsystem und den damit aufgebauten TTS gemäß den Hauptansprüchen. Gegenstand der Unteransprüche sind vorteilhafte Ausführungsformen der erfindungsgemäßen Systeme und Matrizes. Des weiteren umfasst die Erfindung das Verfahren zur Herstellung derartiger Mischsysteme, Matrizes und transdermale therapeutische Systeme.

Es war überraschend und für den Fachmann nicht vorauszusehen und darin liegt die Lösung dieser Aufgaben, dass ein Mischsystem bestehend aus der Kombination von Polyvinylpyrrolidon (PVP) und/oder deren Copolymere und Dimethylsulfoxid (DMSO) die Aufnahme von polaren Wirkstoffen in unpolaren Polymermatrizes ermöglicht und das Auskristallisieren der Wirkstoffe in den unpolaren Polymermatrizes verhindert ohne dass zusätzliche Lösungsmittel eingesetzt werden.

Das erfindungsgemäße Mischsystem umfasst Poly(1-vinyl-2-pyrrolidone) der allgemeinen Formel 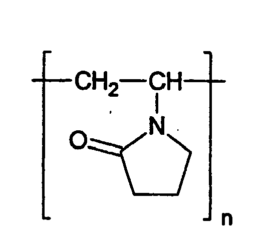 mit n = 350 bis 13500 und/oder deren Copolymere und Dimethylsulfoxid.

Das Gewichtsverhältnis von Poly(1-vinyl-2-pyrrolidone) und/oder deren Copolymere und Dimethylsulfoxid liegt bevorzugt im Bereich von 1:99 bis 99:1, insbesondere 20:80 bis 80:20, insbesondere 50:50.

Als weiter vorteilhaft anzusehen ist, wenn Poly(1-vinyl-2-pyrrolidone) und/oder deren Copolymere ein mittleres Molekulargewicht von 2000 bis 1.500.000 g/mol, insbesondere 44.000 bis 54.000 g/mol aufweisen.

Die Kombination der beiden Verbindungen zu einem Mischsystem erweist sich als überaus vorteilhaft für verschiedenste Einsatzgebiete, wie u.a. als Lösungsvermittler polarer Wirkstoffe in unpolaren Polymermatrizes.

Als Polymermatrizes werden solche Massen bevorzugt, die gänzlich ohne Lösungsmittel auskommen. Besonders bevorzugt sind sog. hot-melt Massen. Heißschmelzklebemassen erlauben die Vermeidung von Nachteilen, die mit dem bei herkömmlichen wirkstoffhaltigen Klebemassen notwendigen Einsatz von Lösemitteln verbunden sind. Als Heißschmelzklebemassen lassen sich vorteilhafterweise thermoplastische Heißschmelzselbstklebemassen auf Basis natürlicher und synthetischer Kautschuke und anderer synthetischer Polymere wie Acrylate, Methacrylate, Polyurethane, Polyolefine, Polyvinylderivate, Polyisobutylene, Polyester oder Silikone einsetzen. Zusatzstoffe wie Klebharze, Weichmacher, Stabilisatoren und andere Hilfsstoffe können soweit erforderlich zugesetzt werden.
Die Vorteile der Heißschmelzklebemassen bei der Herstellung von wirkstoffhaltigen Klebemassen werden schon seit einigen Jahren in der Patentliteratur beschrieben (siehe zum Beispiel EP 0 305 757 A1).

Um die funktionsgerechte Verwendung sicherzustellen, können die Heif3schmelzklebemassen geschäumt sein, und zwar vorzugsweise geschäumt mit inerten Gasen wie Stickstoff, Kohlendioxid, Edelgasen, Kohlenwasserstoffen oder Luft oder deren Gemischen. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.

Der Schäumungsgrad, d.h. der Gasanteil, sollte mindestens etwa 5 Vol.% betragen und kann bis zu etwa 50 Vol.% reichen. In der Praxis haben sich Werte von 10 Vol.% bis 35 Vol.%, bevorzugt 15 Vol.%, gut bewährt. Wird bei relativ hohen Temperaturen von ungefähr 100 °C und vergleichsweise hohem Innendruck gearbeitet, entstehen sehr offenporige Klebstoffschaumschichten, die besonders gut luft- und wasserdampfdurchlässig sind. Ein Schäumungsgrad von über 50 Vol.% wird dabei als technisch unpraktikabel angesehen, da die Kohäsion der Masse sich dadurch verringert. Aus der Vielzahl bekannter Polymermatrizes sind Polyisobutylene besonders bevorzugt. Polyisobutylene erfüllen als Matrixgrundlage die Anforderungen einer selbstklebenden, hautschonenden und schmerzfrei ablösbaren Polymermatrix besonders gut, so dass es folgerichtig ist, die Polyisobutylene bevorzugt als Matrixgrundlage auszuwählen.

Als Polyvinylpyrrolidon (PVP) kann, z.B. Luviskol und Kollidon der Firma BASF, eingesetzt werden, bevorzugt in einer Menge von 0,25 - 60 Gew.%, besonders bevorzugt zwischen 1-30 Gew.%. In gleichem Maße können auch PVP-Copolymerisate wie beispielsweise Vinylpyrrolidon-Vinylacetat (Povidonacetat; Kollidon VA 64), Terpolymere auf Basis Vinylpyrrolidon und Acrylsäure oder Methacrylsäure bzw. deren Ester (Luviflex VBM 35), Copolymerisate aus Vinylpyrrolidon und Vinylimidazoliummethochlorid (Luviquat-Marken) als sog. PVP- agenz eingesetzt werden.

Das System ist insbesondere als Gel funktionsfähig. In anderer Form, z.B. einer Emulsion, lassen sie sich ebenso einsetzen, oder beide Komponenten werden einzeln nacheinander in das System eingearbeitet. Wichtig für die Stabilität der Masse ist dabei, dass beide Komponenten im System vorhanden sind.

In der Kombination der beiden Verbindungen PVP und/oder deren Copolymere und DMSO liegt der nicht vorhersehbare, vorteilhafte Schritt zur Lösung der gestellten Aufgaben. Beide Verbindungen erbringen synergistisch erst den gewünschten Effekt der Integration polarer Wirkstoffe in unpolaren Polymermatrizes. Der Einsatz jeder Verbindung alleine führt nicht zum Erfolg. Dimethylsulfoxid ist zu polar und tritt nach kurzer Lagerzeit wieder aus der Polymermatrix aus. Durch diesen Effekt würde die Klebkraft der Matrix sehr stark absinken, was für eine lange Verweildauer auf der Haut unerwünscht ist. PVP ist in unpolaren Polymeren, insbesondere Polyisobutylen, unlöslich und würde in Form von kugelförmiger Einschlüssen vorliegen. Erst durch die Herstellung des PVP/DMSO-Mischsystems, das vorteilhafterweise als Gel formuliert wird, ist eine vollständige Integration der beiden Komponenten in die unpolare Matrix möglich. Man stellt weder ein Austreten des DMSO aus der Matrix, noch eine Beeinträchtigung der Klebkraft fest. Darüber hinaus kann das PVP und/oder deren Copolymere Koordinationsverbindungen mit Füllstoffen eingehen, die in der Matrix enthalten sind, sofern diese polare Gruppen, wie beispielsweise Hydroxygruppen besitzen. Über die N-Funktionalität des PVP können so Wirk- und/oder Füllstoffe assoziiert werden. Die Assoziation wird durch die guten Lösungseigenschaften des DMSO unterstützt. Damit wird eine synergistische Wirkung der beiden Agentien erzeugt. Zu den assoziierenden Füllstoffen können unter anderem verschiedene Cellulosetypen gewählt werden. Beispielweise lassen sich pulvrige Cellulosen, wie Elcema P020 der Fa. Degussa, als auch mikrokristalline Cellulose verwenden. Durch die Koordination des PVP an die Cellulose wird eine deutlich höhere Härte des Systems gegenüber dem PVP-freien Systemen einstellbar. Dieser Effekt führt zu einer weiteren Verbesserung der Kompatibilität des PVP/DMSO-Mischsystems mit der Polymermatrix.

Ein weiterer wesentlicher Vorteil des erfindungsgemäßen Mischsystems und deren Einsatz in Polymermatrizes ist deren Steuerung verschiedener physikalischer Eigenschaften der Matrix über die eingesetzte Menge des PVP/DMSO-Systems, wie z.B. die Härte oder die Flexibilität der Masse. Zusätzlich lässt sich über den Polymerisationsgrad des PVP die Härte des Systems steuern, wobei ein zu hoher Polymerisationsgrad des PVP härtere Massen zur Folge haben wird. Dadurch ist der Polymerisationsgrad gewissen Einschränkungen unterworfen. Ebenso ist das Klebevermögen bei höherer Hautfeuchte variierbar. Das Pflaster ist okklusiv. PVP nimmt als Filmbildner Wasser auf, so dass die Massen sehr gut auf die Haut aufziehen und stark kleben können. Die bevorzugte Einsatzmenge des erfindungsgemäßen Mischsystems in Polymermatrizes liegt zwischen 2 und 9 Gew.% bezogen auf die Gesamtmasse der Matrix, incl. Wirkstoff.

PVP gilt als weitestgehend inert, so dass nicht mit chemischen Reaktionen mit den pharmazeutischen Wirkstoffen in der Polymermatrix gerechnet werden muss. Im Gegensatz zu dem aus der WO 01/68060 beschriebenen System auf Lösungsmittelbasis und Polyacrylaten ist PVP in einem Hotmelt-Prozess nicht in reiner Form einsetzbar, wenn mit unpolaren Polymeren als Matrix gearbeitet wird. Wie oben beschrieben löst sich das PVP dann nur schlecht in der Matrix. Im Prozess auf Lösungsmittelbasis wird hingegen mit Alkoholen als Lösungsmitteln gearbeitet. Diese Lösungsmittel führen zu einem Quellen des PVPs. Dadurch entfaltet der Rohstoff zwar seine filmbildenden Eigenschaften und kann homogen in die Masse eingearbeitet werden und dieser homogene Zustand bleibt auch nach dem zwingenden Entfernen des Lösungsmittels erhalten, aber die Nachteile von lösungsmittelhaltigen Systemen sind bekannt. Das erfindungsgemäße System kommt ohne zusätzliche Lösemittel aus, da über das DMSO ein Film bzw. Gel ausgebildet wird und das PVP, begünstigt durch das DMSO, als "Film" in der Matrix vorliegt.

Der PVP Film als solches ist bei der Löslichkeit des Wirkstoffs sehr vorteilhaft.

Das erfindungsgemäße Mischsystem aus PVP und/oder deren Copolymere und DMSO hat sehr gute Lösungseigenschaften für polare Wirkstoffe wie beispielsweise Ibuprofen. Die gute Lösungsvermittlung des erfindungsgemäßen Mischsystems für Wirkstoffe in unpolaren Matrizes beruht einerseits darauf, dass saure Wirkstoffe an die Stickstofffunktionen des PVP koordinieren können und dass andererseits das Dimethylsulfoxid im Gel oder Mischsystem ein sehr hohes Löslichkeitspotenzial besitzt. Die Stabilisierung des Wirkstoffes gegenüber einem Auskristallisieren aus der Matrix wird durch beide Eigenschaften synergistisch verstärkt.

Bei Ibuprofen handelt es sich um die (±)-2-(4-Isobutylphenyl)propionsäure (C₁₃H₁₈O₂; M_{R} 206,28; Schmelzpunkt 75 bis 77°C) mit der allgemeinen Formel 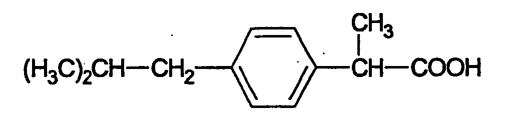 (nach Römpp Lexikon Chemie, Version 1.3, Stuttgart/New York: Georg Thieme Verlag 1997). Ibuprofen ist ein bekannter Wirkstoff mit analgetischen und entzündungshemmenden Eigenschaften und wird deshalb bei Weichteil-Rheuma und entzündlichen Gelenkserkrankungen eingesetzt. Meist wird der Wirkstoff in Form von Tabletten oder Suppositorien verwendet. Orale Verabreichung kann jedoch zu Nebenwirkungen wie Übelkeit, Erbrechen, Schwindeigefühl und Kopfschmerzen führen. Ibuprofen darf aus diesem Grund nicht von Patienten mit Magengeschwüren eingenommen werden.
Nach topischer Applikation erreicht der Wirkstoff das Ziel ohne vorherige Metabolisierung durch die Leber. Dadurch lassen sich Nachteile wie der sog. first-pass effect und Nebenwirkungen vermeiden, die mit der oralen oder rektalen Gabe von Ibuprofen verbunden sind. Die Freisetzung des Wirkstoffs aus einer klebenden Matrix hat gegenüber herkömmlichen topischen Darreichungsformen wie Cremes oder Salben weitere Vorteile. Der Wirkstoff wird kontinuierlich aus der Matrix freigesetzt und nicht in einzelnen Dosen appliziert. Beim Auftreten von unerwünschten Nebenwirkungen kann das wirkstoffhaltige Pflaster einfach entfernt und damit die Abgabe von Ibuprofen unterbrochen werden.
Der Erfindungsgedanke umfasst daher auch wirkstoffhaltige Pflaster mit einem Trägermaterial und einer darauf zumindest partiell aufgebrachten erfindungsgemäßen Heißschmelzklebemasse, die sich dadurch auszeichnen, dass die Heißschmelzklebemasse Ibuprofen enthält.

Vorzugsweise liegen die Mengenkonzentrationen des Ibuprofen in der Heißschmelzklebemasse zwischen 0,01 bis 50 Gew.%, vorzugsweise 0,1 bis 20 Gew.%.

Neben dem oben angeführten Ibuprofen lassen sich mit Hilfe des oben angeführten Systems weitere Wirkstoffe in die Masse einarbeiten. Typische Wirkstoffe in der polymeren Klebemasse sind - ohne den Anspruch der Vollständigkeit im Rahmen der vorliegenden Erfindung zu erheben:

| **Indikation:** | **Wirkstoff** |
|---|---|
| Antimykotika | Nafitin |
| | Amorrolfin |
| | Tolnaftat |
| | Ciclopirox |
| Antiseptika | Thymol |
| | Eugenol |
| | Triclosan |
| | Hexachlorophen |
| | Benzalkoniumchlorid |
| | Clioquinol |
| | Chinolinol |
| | Undecensäure |
| | Ethacridin |
| | Chlorhexidin |
| | Hexetidin |
| | Dodicin |
| | lod |
| Nichtsteroidale Antirheumatika | Glykolsalicylat |
| | Flufenaminsäure |
| | Ibuprofen |
| | Etofenamat |
| | Ketoprofen |
| | Piroxicam |
| | Indomethacin |
| Antipuriginosa | Polidocanol |
| | Isoprenalin |
| | Crotamiton |
| Lokalanästhetika | Benzocain |
| Antipsoriatika | Ammoniumbitumasulfonat |
| Keratolytika | Harnstoff |
| | Salicylsäure |

Daneben können auch hyperämisierende Wirkstoffe wie natürliche Wirkstoffe des Cayenne-Pfeffers oder synthetische Wirkstoffe wie Nonivamid, Nicotinsäurederivate, bevorzugt Benzylnicotinat oder Propylnicotinat, genannt werden. Darüber hinaus ist auch eine Anwendung verschiedener ätherischer Öle möglich.

Ein transdermales therapeutisches System (TTS), zumeist in Form eines Pflasters, umfasst eine erfindungsgemäße selbstklebende wirkstoffhaltige Polymermatrix, eine wirkstoffundurchlässige Rückschicht und eine ablösbare Schutzschicht, die vor dem Applizieren auf der Haut entfernt wird. Weitere Ingredienzien, wie Füllstoffe, Stabilisatoren, Enhancer und/oder kosmetische Zusätze können in der Polymermatrix eingebaut werden, um das TTS an die unterschiedlichen Anwendungsgebiete anzupassen und um ein anwendungsfreundliches TTS bereit zu stellen. Die erfindungsgemäße Polymermatrix kann selbstklebend oder nicht klebend ausgeführt sein. In letzterem Fall, beispielsweise einem sog. Island Dressing, sollten jedoch Hilfsmittel für die Befestigung auf der Haut gegeben sein.
Besonders vorteilhaft ist die erfindungsgemäße Polymermatrix auf einer flexiblen Deckschicht aufgebracht, insbesondere bei der Verwendung als selbstklebendes Pflaster.

Solche transdermalen therapeutischen Systeme erhalten durch die erfindungsgemäße Polymermatrix verschiedene Vorteile. So wird beispielsweise eine sehr gleichmäßige Wirkstoffabgabe über einen Zeitraum von bis zu 24 Stunden gewährleistet. Die mechanischen Eigenschaften des Pflasters, u. a. die Flexibilität und die haftklebrigen Eigenschaften können über die Menge an PVP/DMSO Gel in hohem Maße beeinflusst werden. Das Pflaster nimmt Feuchtigkeit auf, wodurch ein Feuchtigkeitsfilm zwischen Klebmatrix und Haut verhindert wird, der eine zusätzliche Barriere für die Aufnahme des Wirkstoffs in die Haut darstellen würde. Die Matrix behält durch die Flüssigkeitsaufnahme auch bei vermehrter Schweissbildung ihre haftklebrigen Eigenschaften bei. Des weiteren kann die Matrix mit Wirkstoff übersättigt werden, ohne dass ein Auskristallisieren stattfindet.

Aufgebaut ist ein entsprechendes Pflaster aus einem Träger wie Folien, Vliese, Gewebe, Schäume etc., der Klebmatrix und Abdeckfolie, Abdeckpapier oder Trennpapier zum Schutz der klebenden Matrix vor dem Gebrauch des Pflasters.
In einer weiteren bevorzugten Ausführungsform der Erfindung werden als Träger Polymerfolien, Vliese, Gewebe sowie deren Kombinationen eingesetzt. Als Trägermaterialien stehen u.a. Polymere wie Polyethylen, Polypropylen und Polyurethan oder auch Naturfasern zur Auswahl.
Zusammenfassend kann festgehalten werden, dass als Trägermaterialien sich alle starren und elastischen Flächengebilde aus synthetischen und natürlichen Rohstoffen eignen. Bevorzugt sind Trägermaterialien, die so eingesetzt werden können, dass sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona und Hydrophobieren; geläufige Nachbehandlungen sind Kalandern, Tempern, Kaschieren, Stanzen und Eindecken. Besonders vorteilhaft ist, wenn das Trägermaterial sterilisierbar, bevorzugt γ-(gamma) sterilisierbar, ist.
Die genannten Eigenschaften der Klebmatrix legen insbesondere die Verwendung für medizinische Produkte, insbesondere Pflaster, medizinische Fixierungen, Wundabdeckungen, orthopädische oder phlebologische Bandagen und Binden nahe.

Schließlich kann die Polymermatrix mit einem klebstoffabweisenden Trägermaterial, wie silikonisiertem Papier, eingedeckt oder mit einer Wundauflage oder einer Polsterung versehen werden. Auf seiner selbstklebend ausgerüsteten, später der Haut zugewandten Seite ist das erfindungsgemäße Pflaster über seine ganze Breite bis zum Gebrauch üblicherweise mit einem klebstoffabweisenden Trägermaterial abgedeckt. Dieses schützt die Selbstklebeschicht aus der gut hautverträglichen Klebemasse der Matrix, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.

Weitere Ausführungsformen können dergestalt sein, dass das TTS einschichtig oder mehrschichtig selbstklebend aufgebaut ist. Weiterhin kann zwischen der Rückseite der Matrix und dem Abdeckträger sich eine zweite erfindungsgemäße Polymermatrix mit höherer Wirkstofflöslichkeit als Reservoir befinden.
Auf der Klebseite der Matrix befindet sich teilweise (z.B. am Rand) eine zweite Matrix mit hoher Klebkraft zur zusätzlichen Fixierung, aber ungenügender bzw. unnötiger Wirkstofflöslichkeit.
Die wirkstofffreie Matrix befindet sich zwischen zwei nicht verankernden Folien und wird zur Fixierung von Elektroden etc., oder wegen der Wasseraufnahmefähigkeit bei entsprechender Geometrie von Colostomie-/Ileostomiebeuteln genutzt.

Die Herstellung der erfindungsgemäßen Polymermassen erfolgt lösungsmittelfrei. Sowohl in einem diskontinuierlichen Prozess in einem Kneter als auch kontinuierlich in einem Extruder lassen sich die Polymermassen herstellen. Bei der Herstellung im Kneter wird das hochpolymere Material vorgelegt. Das Material wird beispielsweise bei 20 U/min bei 50,0°C vorgeknetet. Anschließend werden die übrigen Materialien nacheinander zudosiert. Zwischen jeder Zugabe wird die Masse zwischen 15min und 60min homogenisiert, wobei die flüssigen Komponenten und der Wirkstoff am Ende zugegeben werden. Nach dem Ende des Knetvorgangs wird die Masse aus dem Kneter ausgetragen und kann beschichtet werden.
Bei der Extrusion der Masse wird zunächst das hochpolymere Material über die Einzugszone zudosiert und aufgeschlossen. Anschließend werden die übrigen Komponenten der Reihe nach zudosiert, wobei auch hier zunächst die festen und anschließend die flüssigen Komponenten zugegeben werden. Die Zudosierung des Wirkstoffs erfolgt wieder als letzte Komponente. Im Gegensatz zum Knetprozess wird bei der Extrusion Inline beschichtet.

Die nachfolgenden Beispiele veranschaulichen die Erfindung ohne sie einzuschränken. In der nachfolgenden Tabelle sind die erfindungsgemäßen Mischsysteme, Polymermatrizes und TTS sowie deren Herstellung aufgeführt.
In den aufgeführten Beispielen ist die Wirkstofffreisetzung aus der erfindungsgemäßen Matrix auf einer Silikonmembran aufgeführt.

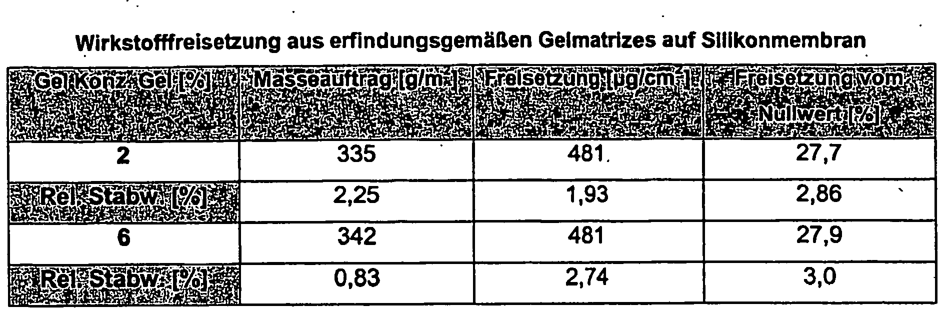

### Beispiel 1

In einem Becherglas werden 20.0g (50% w/w) Dimethylsulfoxid vorgelegt und auf 70°C erwärmt. Unter Rühren werden 20.0g (50% w/w) Kollidon 30^{®} hinzugefügt. Die Mischung wird 60min bei 70°C gerührt. Die fertige Mischung liegt als Gel vor, wird abgekühlt und in ein Vorratsgefäß abgefüllt.

### Beispiel 2

In einem Becherglas werden 20,0g (50% w/w) Dimethylsulfoxid vorgelegt und auf 70°C erwärmt. Unter Rühren werden 20,0g (50% w/w) Kollidon 90^{®} hinzugefügt. Die Mischung wird 60min bei 70°C gerührt. Die fertige Mischung liegt als Gel vor, wird abgekühlt und in ein Vorratsgefäß abgefüllt.

### Beispiel 3

In einem IKA Meßkneter werden 50,35g (26,5% w/w) Vistanex LM MH, 22,80g (12,0% w/w) Vistanex MM L80 und 21,85g (11,5% w/w) Eastoflex PLS E1003D bei 70°C 15min geknetet. Es werden 60,80g (32,0% w/w) pulvrige Cellulose hinzugefügt und 60min geknetet. Anschließend fügt man 13,30g (7,0% w/w) Cetiol V und 11,40g (6,0% w/w) DMSO/PVP-Mischung hinzu. Die Mischung wird 5 min geknetet, dann erfolgt die Zugabe von 9,50g (5,0% w/w) Ibuprofen. Die resultierende Mischung wird weitere 60min bei 80-100°C geknetet.
Die fertige Masse wird dann zwischen Trennpapier mit einer Hydraulikpresse ausgepresst und anschließend auf Träger und Trennfolie umkaschiert.

### Beispiel 4

In einem IKA Meßkneter werden 49,40g (26,0% w/w) Vistanex LM MH, 20,90g (11,0% w/w) Vistanex MM L80 und 26,60g (14,0% w/w) Eastoflex PLS E1003D bei 70°C 15min geknetet Es werden 60,80g (32,0% w/w) pulvrige Cellulose hinzugefügt und 60min geknetet. Anschließend fügt man 19,00g (10,0% w/w) Cetiol V und 11,40g (2,0% w/w) DMSO/PVP-Mischung hinzu. Die Mischung wird 5 min geknetet, dann erfolgt die Zugabe von 9,50g (5,0% w/w) Ibuprofen. Die resultierende Mischung wird weitere 60min bei 80 -100°C geknetet. Die fertige Masse wird dann zwischen Trennpapier mit einer Hydraulikpresse ausgepresst und anschließend auf Träger und Trennfolie umkaschiert.

### Beispiel 5

In einem IKA Meßkneter werden 41,80g (22,0% w/w) Vistanex LM MH, 35,15g (18,5% w/w) Vistanex MM L80 und 27,51g (14,5% w/w) Eastoflex PLS E1003D bei 70°C 15min geknetet. Es werden 62,70g (33,0% w/w) pulvrige Cellulose hinzugefügt und 60min geknetet. Anschließend fügt man 19,00g (10,0% w/w) Cetiol V und 11,40g (2,0% w/w) DMSO/PVP-Mischung hinzu. Die Mischung wird 5 min geknetet, dann erfolgt die Zugabe von 0,095g (0,05% w/w) Capsaicin. Die resultierende Mischung wird weitere 60min bei 80-100°C geknetet.
Die fertige Masse wird dann zwischen Trennpapier mit einer Hydraulikpresse ausgepresst und anschließend auf Träger und Trennfolie umkaschiert.

### Beispiel 6

In einem IKA Meßkneter werden 46,55g (24,5% w/w) Vistanex LM MH, 26,60g (14,0% w/w) Vistanex MM L80 und 22,80g (12,0% w/w) Eastoflex PLS E1003D bei 70°C 15min geknetet. Es werden 62,70g (33,0% w/w) pulvrige Cellulose hinzugefügt und 60min geknetet Anschließend fügt man 19,00g (10,0% w/w) Eutanol G und 11,40g (6,0% w/w) DMSO/PVP-Mischung hinzu. Die Mischung wird 5 min geknetet, dann erfolgt die Zugabe von 0,95g (0,5% w/w) Piroxicam. Die resultierende Mischung wird weitere 60min bei 80 -100°C geknetet.
Die fertige Masse wird dann zwischen Trennpapier mit einer Hydraulikpresse ausgepresst und anschließend auf Träger und Trennfolie umkaschiert.

### Beispiel 7 (Extrusion)

Die Herstellung der Masse erfolgt in einem 50mm Leistritz Doppelschneckenextruder mit einem Masseausstoss von 40kg/h. Das Temperaturprofil für den Prozeß beträgt 130 - 100°C von der Aufgabezone bis zur Austragszone. Die Inhaltsstoffe der Klebmatrix werden nacheinander entlang der Gesamtlänge des Verfahrensteils in folgender Reihenfolge zudosiert:

| | | | |
|---|---|---|---|
| 1. | Vistanex MM L80: | 4,4kglh | Feststoff granuliert |
| 2. | Vistanex LM MH: | 10,4kg/h | Schmelze |
| 3. | Eastoflex PLS E1003D: | 5,6kg/h | Schmelze |
| 4. | pulvrige Cellulose: | 12,8kg/h | Feststoff |
| 5. | DMSO/PVP Mischung | 0,8kg/h | flüssig |
| 6. | Cetiol V | 4.0kg/h | flüssig |
| 7. | Ibuprofen | 2.0kg/h | Feststoff |

Die Masse wird kontinuierlich hergestellt und aus einer Breitschlitzdüse ausgetragen. Zwischen Austragszone und Breitschlitzdüse ist eine Zahnradpumpe installiert, die für einen gleichmäßigen Masseaustrag sorgt. Die Klebmasse wird anschließend über einen Kalander zwischen Trägermaterial und einer Trennfolie beschichtet und zur weiteren Verarbeitung auf Rollen aufgewickelt.

### Bei den zitierten Stoffen handelt es sich um:

| Name | Ingredienz |
|---|---|
| Kollidon 30^{®} | Polyvinylpyrrolidon MW: 44.000 bis 54.000 |
| Kollidon 90^{®} | Polyvinylpyrrolidon MW 1.000.000 bis 1.500.000 |
| Vistanex LM MH | niedermolekulares Polyisobutylen MW: 12.000 - 53.000 |
| Vistanex MM L80 | hochmolekulares Polyisobutylen MW: 750.000 -1.050.000 |
| Eastoflex PLS E1003D | amorphes α-Polyolefin |
| Cetiol V | Decyloleat |
| Eutanol G 1 | Oktyldodecanol |
| Cellulose Elcema P020 | pulvrige Cellulose |
| Avicel PH 103 | miktokrist. Cellulose |

Die Matrizes in den oben angeführten Beispielen zeigen alle den Vorteil, dass der Wirkstoff nicht auskristallisiert. Verwendet man Matrizes die kein DMSO/PVP-Gel enthalten, so findet innerhalb von 1 bis 5 Tagen eine vollständige Kristallisation des Wirkstoffs statt. Dieses führt zu einer Abnahme der Wirkstofffreisetzung der Matrix um bis zu 90%. Die Einarbeitung des Gels führt darüber hinaus noch zu einer Veränderung der mechanischen Eigenschaften der Matrix. Mit zunehmendem Gelanteil werden die Matrizes härter, so dass sich die Fließeigenschaften der Matrix in gewissen Grenzen beeinflussen lassen. Ebenfalls wird die Wirkstoffpermeation durch die Haut mit dem Einsatz des DMSO/PVP-Gels verbessert.

## Patentansprüche

1. Polymermatrix umfassend
- eine Polymermatrix, deren Polymere einen hydrophoben Charakter aufweisen,
- 0,1 bis 25 Gew.%, bezogen auf die Masse der Gesamtpolymermatrix, eines Mischsystems umfassend Poly(1-vinyl-2-pyrrolidone) der allgemeinen Formel 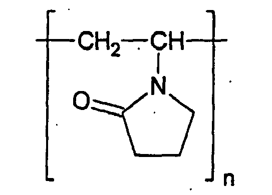 mit n = 350 bis 13500 und/oder deren Copolymere und Dimethylsulfoxid
im Gewichtsverhältnis von 20:80 bis 80:20 sowie
- einen oder mehrere polare pharmazeutische Wirkstoffe,
- und keine zusätzlichen Lösemittel

2. Polymermatrix nach Anspruch 1, **dadurch gekennzeichnet, dass** das Mischsystem als Gel vorliegt.

3. Polymermatrix nach Anspruch 1 oder 2, **dadurch gekennzeichnet, dass** Poly(1-vinyl-2-pyrrolidone) und/oder deren Copolymere und Dimethylsulfoxid im Gewichtsverhältnis von 50:50 vorliegen.

4. Polymermatrix nach einem der vorstehenden Ansprüche, **dadurch gekennzeichnet, dass** Poly(1-vinyl-2-pyrrolidone) und/oder deren Copolymere mit einem mittleren Molekulargewicht von 2000 bis 1.500.000 g/mol, insbesondere 44.000 - 54.000 g/mol vorliegen.

5. Polymermatrix nach einem der vorstehenden Ansprüche, **dadurch gekennzeichnet, dass** die Matrix eine Heißschmelzklebemasse ist.

6. Polymermatrix nach Anspruch 5, **dadurch gekennzeichnet, dass** die Matrix als hydrophobe Basispolymere Polyisobutylen enthält.

7. Polymermatrix nach einem der vorstehenden Ansprüche enthaltend einen oder mehrere Füllstoffe.

8. Polymermatrix nach Anspruch 7, **dadurch gekennzeichnet, dass** als Füllstoff Cellulose eingesetzt wird.

9. Polymermatrix nach einem der vorstehenden Ansprüche enthaltend als Wirkstoff Ibuprofen, Nafitin, Amorrolfin, Tolnaftat, Ciclopirox, Thymol, Eugenol, Triclosan, Hexachlorophen, Benzalkoniumchlorid, Clioquinol, Chinolinol, Undecensäure, Ethacridin, Chlorhexidin, Hexetidin, Dodicin, Iod, Glykolsalicylat, Flufenaminsäure, Etofenamat, Ketoprofen, Piroxicam, Indomethacin, Polidocanol, Isoprenalin, Crotamiton, Benzocain, Ammoniumbitumasulfonat, Harnstoff und/oder Salicylsäure.

10. Polymermatrix nach Anspruch 9, **dadurch gekennzeichnet, dass** Ibuprofen als Wirkstoff ausgewählt wird und mit einem Anteil von 0,01 bis 50 Gew.%, vorzugsweise 0,1 bis 20 Gew.%, bezogen auf die polymere Gesamtmasse vorliegt.

11. Polymermatrix nach einem der vorstehenden Ansprüche, **dadurch gekennzeichnet, dass** das Mischsystem in einem Anteil von 2 bis 9 Gew.% bezogen auf die Masse der Gesamtpolymermatrix, enthalten ist.

12. Verwendung der Polymermatrix nach einem der vorstehenden Ansprüche in einem Transdermales Therapeutisches System, insbesondere Pflaster oder Bandage.

## Claims

1. Polymer matrix comprising
- a polymer matrix whose polymers have a hydrophobic character,
- from 0.1 to 25% by weight, based on the mass of the total polymer matrix, of a hybrid system comprising poly(1-vinyl-2-pyrrolidones) of the general formula 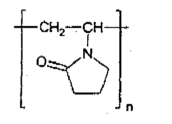 with n = 350 to 13 500 and/or copolymers thereof and dimethyl sulfoxide
in a weight ratio of from 20:80 to 80:20, and
- one or more polar pharmaceutically active substances,
- and no additional solvents.

2. Polymer matrix according to Claim 1, **characterized in that** the hybrid system is in the form of a gel.

3. Polymer matrix according to Claim 1 or 2, **characterized in that** poly(1-vinyl-2-pyrrolidones) and/or copolymers thereof and dimethyl sulfoxide are in a weight ratio of 50:50.

4. Polymer matrix according to one of the above claims, **characterized in that** poly(1-vinyl-2-pyrrolidones) and/or copolymers thereof with an average molecular weight of from 2000 to 1 500 000 g/mol, in particular 44 000 - 54 000 g/mol, are present.

5. Polymer matrix according to one of the above claims, **characterized in that** the matrix is a hotmelt adhesive.

6. Polymer matrix according to Claim 5, **characterized in that** the matrix comprises polyisobutylene as hydrophobic base polymer.

7. Polymer matrix according to one of the above claims comprising one or more fillers.

8. Polymer matrix according to Claim 7, **characterized in that** cellulose is employed as filler.

9. Polymer matrix according to one of the above claims comprising as active substance ibuprofen, nafitine, amorrolfine, tolnaftate, ciclopirox, thymol, eugenol, triclosan, hexachlorophene, benzalkonium chloride, clioquinol, quinolinol, undecenoic acid, ethacridine, chlorhexidine, hexetidine, dodicine, iodine, glycol salicylate, flufenamic acid, etofenamate, ketoprofen, piroxicam, indomethacin, polidocanol, isoprenaline, crotamiton, benzocaine, ammonium bitumasulfonate, urea and/or salicylic acid.

10. Polymer matrix according to Claim 9, **characterized in that** ibuprofen is selected as active substance and is present with a proportion of from 0.01% to 50% by weight, preferably from 0.1% to 20% by weight, based on the polymeric total composition.

11. Polymer matrix according to one of the above claims, **characterized in that** the hybrid system is present in a proportion of from 2% to 9% by weight, based on the mass of the total polymer matrix.

12. Use of Polymer matrix according to one of the above claims in a transdermal therapeutic system, especially a patch or bandage.

## Revendications

1. Matrice polymère, comprenant
- une matrice polymère, dont les polymères présentent un caractère hydrophobe,
- 0,1 à 25% en poids, par rapport à la masse de la matrice polymère totale, d'un système mixte comprenant de la poly(1-vinyl-2-pyrrolidone) de formule générale 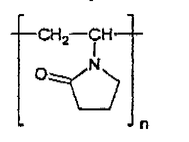 avec n = 350 à 13 500 et/ou ses copolymères et du diméthylsulfoxyde dans un rapport pondéral de 20:80 à 80:20 ainsi que
- une ou plusieurs substances actives pharmaceutiques polaires,
- et ne comprenant pas de solvants supplémentaires.

2. Matrice polymère selon la revendication 1, **caractérisée en ce que** le système mixte se trouve sous forme de gel.

3. Matrice polymère selon la revendication 1 ou 2, **caractérisée en ce que** la poly(1-vinyl-2-pyrrolidone) et/ou ses copolymères et le diméthylsulfoxyde se trouvent dans un rapport pondéral de 50:50.

4. Matrice polymère selon l'une quelconque des revendications précédentes, **caractérisée en ce que** la poly(1-vinyl-2-pyrrolidone) et/ou ses copolymères se trouvent avec un poids moléculaire moyen de 2000 à 1 500 000 g/mole, en particulier de 44 000-54 000 g/mole.

5. Matrice polymère selon l'une quelconque des revendications précédentes, **caractérisée en ce que** la matrice est une masse adhésive fondue à chaud.

6. Matrice polymère selon la revendication 5, **caractérisée en ce que** la matrice contient du polyisobutylène comme polymère de base hydrophobe.

7. Matrice polymère selon l'une quelconque des revendications précédentes, contenant une ou plusieurs charges.

8. Matrice polymère selon la revendication 7, **caractérisée en ce que** de la cellulose est utilisée comme charge.

9. Matrice polymère selon l'une quelconque des revendications précédentes, contenant comme substance active de l'ibuprofène, de la nafitine, de l'amorolfine, du tolnaftate, du ciclopirox, du thymol, de l'eugénol, du triclosan, de l'hexachlorophène, du chlorure de benzalconium, du clioquinol, du quinolinol, de l'acide undécénoïque, de l'éthacridine, de la chlorhexidine, de l'hexétidine, de la dodicine, de l'iode, du salicylate de glycol, de l'acide flufénamique, de l'étofénamate, du cétoprofène, du piroxicam, de l'indométhacine, du polidocanol, de l'isoprénaline, du crotamiton, de la benzocaïne, du bituminosulfonate d'ammonium, de l'urée et/ou de l'acide salicylique.

10. Matrice polymère selon la revendication 9, **caractérisée en ce que** l'ibuprofène est choisi comme substance active et se trouve en une proportion de 0,01 à 50% en poids, de préférence de 0,1 à 20% en poids, par rapport à la masse totale polymère.

11. Matrice polymère selon l'une quelconque des revendications précédentes, **caractérisée en ce que** le système mixte est contenu en une proportion de 2 à 9% en poids par rapport à la masse de la matrice polymère totale.

12. Utilisation de la matrice polymère selon l'une quelconque des revendications précédentes dans un système thérapeutique transdermique, en particulier un emplâtre ou un bandage.
